# EUROPEAN PATENT APPLICATION

(11) **EP 4 681 623 A1**
(43) Date of publication of application: **21.01.2026**
(21) Application number: 24770798.7
(22) Date of filing: 08.03.2024
(51) Int. Cl.: A61B 5/02

(54) **PULSE WAVE SENSOR**

(30) Priority: 14.03.2023 JP 2023040008
(71) Applicant: Minebea Mitsumi Inc., Kitasaku-gun, Nagano 3890293 (JP)
(72) Inventor: HARADA, Koshi, Kitasaku-gun, Nagano 389-0293 (JP); TOKUDA, Yuta, Kitasaku-gun, Nagano 389-0293 (JP)
(74) Representative: Winter, Brandl - Partnerschaft mbB
(86) International application number: PCT/JP2024/009160
(87) International publication number: WO 2024/190692

(57) **Abstract**

A pulse wave sensor includes a strain generating body including a first surface and a second surface located on an opposite side of the first surface, a plurality of beams provided on the strain generating body and intersecting each other, one or more load portions disposed in respective regions where the beams intersect each other on the first surface, a pair of strain gauges disposed, on the second surface, on a first beam of the beams, with the load portion interposed between the strain gauges in a plan view, and another pair of strain gauges disposed, on the second surface, on a second beam intersecting the first beam, with the load portion interposed between the strain gauges of the other pair in a plan view. A pulse wave is detected based on a change in resistance value of each of the strain gauges caused by deformation of the beams.

## Description

### TECHNICAL FIELD

The present invention relates to a pulse wave sensor.

### BACKGROUND ART

A pulse wave sensor for detecting arterial pulses generated when the heart pumps blood is known. An example of the pulse wave sensor is a pulse wave sensor provided with a pressure receiving plate as a strain generating body supported in a deflective manner by an action of an external force and a piezoelectric conversion element for converting the deflection of the pressure receiving plate into an electric signal. The pulse wave sensor has a dome-like shape in which a deflective region of the pressure receiving plate becomes a convex surface curved outward, and a pressure detecting element is provided on the inner surface of the top portion of the pressure receiving plate as the piezoelectric conversion element (for example, Patent Document 1).

### RELATED ART DOCUMENTS

### PATENT DOCUMENT

[Patent Document 1] Japanese Unexamined Patent Application Publication No. 2002-78689

### SUMMARY OF THE INVENTION

### PROBLEMS TO BE SOLVED BY THE INVENTION

Since a pulse wave sensor needs to detect low-level signals, a detection portion needs to be reliably disposed in the vicinity of the radial artery or the like of a subject. However, since it is difficult to position a detection portion of the pulse wave sensor at the radial artery or the like of a subject, there are problems that a time required until the pulse wave sensor starts acquiring a pulse wave after the pulse wave sensor is worn becomes longer and that measurement accuracy is degraded due to a positional deviation.

The present invention has been made in view of the above circumstances, and an object of the present invention is to shorten the time for attaching a pulse wave sensor to a subject and improve measurement accuracy.

### MEANS FOR SOLVING THE PROBLEM

According to one aspect of the present disclosure, a pulse wave sensor includes a strain generating body including a first surface and a second surface located on an opposite side of the first surface, a plurality of beams provided on the strain generating body and intersecting each other, one or more load portions disposed in respective regions where the beams intersect each other on the first surface, a pair of strain gauges disposed, on the second surface, on one of the beams, with the load portion interposed between the strain gauges in a plan view, and another pair of strain gauges disposed, on the second surface, on an other beam intersecting the beam on which the pair of strain gauges are disposed, with the load portion interposed between the strain gauges of the other pair in a plan view. A pulse wave is detected based on a change in resistance value of each of the strain gauges caused by deformation of the beams.

### EFFECTS OF THE INVENTION

According to the disclosed technique, it is possible to shorten a time for attaching a pulse wave sensor to a subject and improve measurement accuracy.

### BRIEF DESCRIPTION OF THE DRAWINGS

[FIG. 1] FIG. 1 is a plan view illustrating a pulse wave sensor according to a first embodiment.
[FIG. 2] FIG. 2 is a cross-sectional view illustrating the pulse wave sensor according to the first embodiment.
[FIG. 3] FIG. 3 illustrates a simulation result (1) of an amount of strain in a beam caused by pressing each load portion.
[FIG. 4] FIG. 3 illustrates a simulation result (2) of an amount of strain in a beam caused by pressing each load portion.
[FIG. 5] FIG. 5 is a diagram illustrating a bridge connection.
[FIG. 6] FIG. 6 is a plan view illustrating a strain gauge according to the first embodiment.
[FIG. 7] FIG. 7 is a cross-sectional view (1) illustrating the strain gauge according to the first embodiment.
[FIG. 8] FIG. 8 is a cross-sectional view (2) illustrating the strain gauge according to the first embodiment.

### DESCRIPTION OF EMBODIMENTS

Hereinafter, embodiments of the present invention will be described with reference to the drawings. In the drawings, the same components may be denoted by the same reference numerals. In the drawings, an X direction, a Y direction, and a Z direction that are perpendicular to each other may be defined. In this case, in the X direction, a start point (root) side of the arrow may be referred to as the "-X side", and an end point (arrowhead) side of the arrow may be referred to as the "+X side". The same applies to the Y direction and the Z direction. In the description of the drawings, the same components as those already described need not be described.

### <First Embodiment>

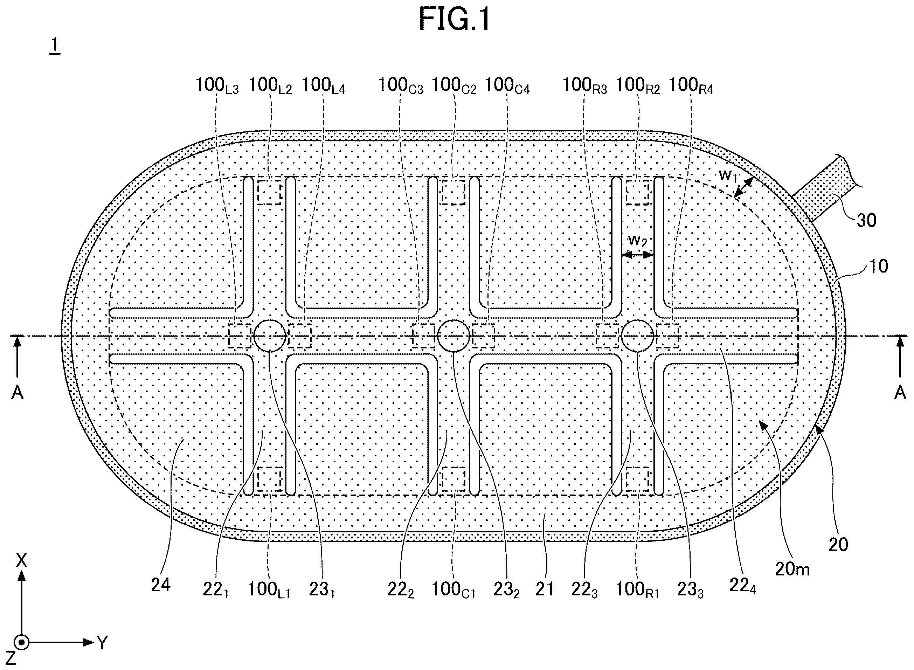

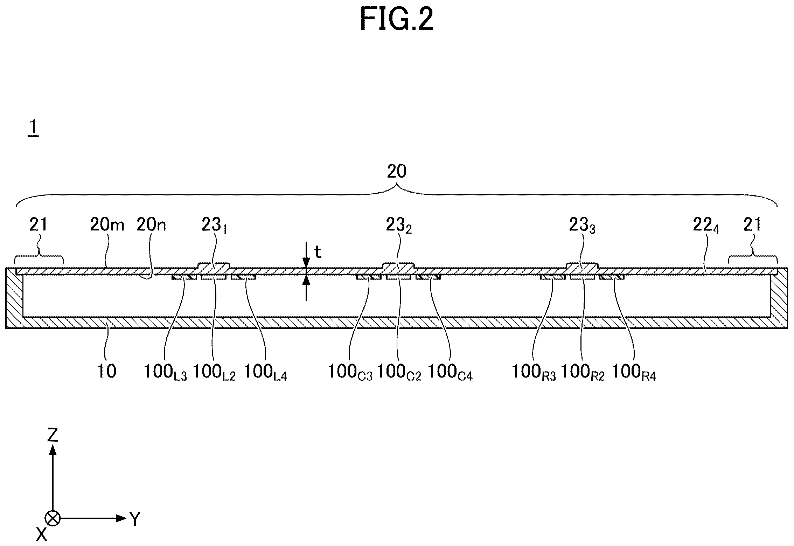

FIG. 1 is a plan view illustrating a pulse wave sensor according to the first embodiment. FIG. 2 is a cross-sectional view illustrating the pulse wave sensor according to the first embodiment, and illustrates a cross section taken along line A-A in FIG. 1.

As illustrated in FIGS. 1 and 2, the pulse wave sensor 1 includes a housing 10, a strain generating body 20, a wire rod 30, and a plurality of strain gauges (100_{L1}, 100_{L2}, 100_{L3}, 100_{L4}, 100_{C1}, 100_{C2}, 100_{C3}, 100_{C4}, 100_{R1}, 100_{R2}, 100_{R3}, and 100_{R4}). When it is unnecessary to distinguish the strain gauges from each other, the strain gauges may be collectively referred to as a "strain gauge 100".

The strain generating body 20 includes a base portion 21, beams 22₁, 22₂, 22₃, and 22₄, load portions 23₁, 23₂, and 23₃, and a plurality of extension portions 24. When it is unnecessary to distinguish the beams from each other, the beams may be collectively referred to as a "beam 22". When it is unnecessary to distinguish the load portions from each other, the load portions may be collectively referred to as a "load portion 23".

The strain generating body 20 has a flat plate shape. The strain generating body 20 includes a first surface 20m and a second surface 20n located on the opposite side of the first surface 20m. The strain generating body 20 has, for example, a shape of twofold symmetry in a plan view. The strain generating body 20 has, for example, a shape in which the Y direction is the longitudinal direction and the X direction is the lateral direction. Specifically, the strain generating body 20 may be formed in an elliptical shape, a rectangular shape, a rounded rectangular shape, or the like. In the illustrated example, the strain generating body 20 has a rounded rectangular shape with the Y direction as the longitudinal direction. The rounded rectangle is a figure having a shape in which some or all of the corners of a rectangle are rounded. The rounded rectangle may be a shape formed by two parallel lines having the same length and semicircles disposed at both ends of the parallel lines. In the case where the strain generating body 20 is formed in an elliptical shape or a rounded rectangular shape, the strain generating body 20 can be reduced in size in the X direction as compared with the case of a circular shape, when the length in the Y direction is the same. If necessary, the strain generating body 20 may be formed in a circular shape, a square shape, or the like in a plan view.

As a material of the strain generating body 20, for example, metal, ceramic, glass, or the like can be used. Examples of the metal used as the material of the strain generating body 20 include stainless steel (SUS), copper, and aluminum. The strain generating body 20 can be integrally formed by, for example, a press working method or the like. The thickness t of the strain generating body 20 excluding the load portion 23 is constant. The thickness t of the strain generating body 20 excluding the load portion 23 may be, for example, equal to or greater than 0.03 mm and equal to and less than 0.3 mm.

In the present embodiment, for convenience, in the pulse wave sensor 1, the side on which the load portion 23 of the strain generating body 20 is provided is referred to as the "upper side", and the side on which the load portion 23 of the strain generating body 20 is not provided is referred to as the "lower side". A surface located on the upper side of each portion is referred to as an "upper surface", and a surface located on the lower side of each portion is referred to as a "lower surface". The pulse wave sensor 1 may be used in an upside-down state. The pulse wave sensor 1 may be disposed at any angle. A "plan view" refers to viewing an object in a normal direction, from the upper side to the lower side with respect to the first surface 20m of the strain generating body 20. A "planar shape" refers to the shape of the object when the object is viewed in the normal direction.

In the pulse wave sensor 1, the housing 10 is a portion that holds the strain generating body 20. The housing 10 is hollow, and the lower surface side is closed and the upper surface side is open. The housing 10 can be formed of, for example, metal, resin, or the like. The strain generating body 20 is fixed by an adhesive or the like so as to close the opening on the upper surface side of the housing 10.

In the strain generating body 20, the base portion 21 is a region having a predetermined width in the outer edge portion of the strain generating body 20. Specifically, the base portion 21 is an annular region outside the broken line illustrated in FIG. 1. The width w₁ of the base portion 21 is equal to or greater than 1 mm and equal to or less than 5 mm, for example. The width w₁ of the base portion 21 may be constant or not constant. The length of the inner side of the base portion 21 in the X direction is, for example, equal to or greater than 5 mm and equal to or less than 35 mm. The length of the inner side of the base portion 21 in the Y direction is greater than the length of the inner side of the base portion 21 in the X direction.

The strain generating body 20 is provided with a plurality of beams 22 intersecting each other. The plurality of beams 22 may include one beam and a plurality of beams intersecting the one beam. The plurality of beams 22 may be perpendicular to each other. Each beam 22 is provided so as to bridge the inside of the base portion 21.

In the illustrated example, the plurality of beams 22 include one linear beam 22₄ extending in the Y direction, and linear beams 22₁, 22₂, and 22₃ extending in the X direction perpendicular to the beam 22₄. The beam 22₄ is longer than the beams 22₁, 22₂, and 22₃. In the illustrated example, the beams 22₁, 22₂, and 22₃ intersecting one beam 22₄ are arranged at equal intervals from each other. The plurality of beams 22 may include four or more beams arranged at equal intervals.

In the beams 22₁, 22₂, 22₃, and 22₄, the width w₂ of the regions other than the intersecting regions are constant, and is, for example, equal to or greater than 1 mm and equal to or less than 5 mm. Although it is not essential that the width w₂ is constant, it is preferable that the width w₂ be constant because a strain can be linearly detected.

It suffices that the plurality of beams 22 include one beam and a plurality of beams intersecting the one beam. One beam or a plurality of beams intersecting one beam need not be linear. The number of the plurality of beams intersecting one beam may be two or more.

In the first surface 20m of the strain generating body 20, the load portion 23 is disposed in a region where the beams 22 intersect. In the illustrated example, the load portion 23₁ is disposed in a region where the beams 22₁ and 22₄ intersect in the first surface 20m of the strain generating body 20. The load portion 23₂ is disposed in a region where the beam 22₂ and the beam 22₄ intersect. The load portion 23₃ is disposed in a region where the beam 22₃ and the beam 22₄ intersect.

In the strain generating body 20, since the plurality of load portions 23 are disposed on one beam 22₄, the load portions 23 influence each other. In other words, when one load portion 23 is pressed, distortion is also generated around the other load portions 23. Even in the case where any of the load portions 23 is pressed, the sum of strains generated around each of the load portions 23 is constant.

The load portion 23 protrudes from the upper surface of the beam 22. An amount of protrusion of the load portion 23 with respect to the upper surface of the beam 22 is, for example, about 0.1 mm. The amount of protrusion of each load portion 23 may be the same or need not be the same. The beam 22 has flexibility and is elastically deformed when a load is applied to the load portion 23.

The planar shape of the load portion 23 is, for example, a circular shape, but may be an elliptical shape or the like. In the case where the planar shape of the load portion 23 is circular, the diameter can be, for example, equal to or greater than 1.5 mm and equal to or less than 2.5 mm. In the case where the planar shape of the load portion 23 is an ellipse, the major axis direction is preferably directed in the Y direction from the viewpoint of making it easy for the load portion 23 to come into contact with the radial artery.

A pitch of the adjacent load portions 23 may be, for example, about equal to or greater than 4 mm and equal to or less than 6 mm. Intervals of the adjacent load portions 23 may be, for example, about equal to or greater than 1 mm and equal to or less than 3 mm. The intervals between the adjacent load portions 23 are preferably equal to or less than the thickness of the radial artery. It is generally said that 80% of Japanese women have a radial artery with a thickness of 2.4 mm or more and 3.0 mm at the maximum, and 80% of Japanese men have a radial artery with a thickness of 2.6 mm or more and 4.0 mm at the maximum. In other words, 80% of Japanese patients have radial arteries with a thickness of 2.4 mm or more and 4.0 mm or less.

Therefore, the intervals between the adjacent load portions are preferably designed to be equal to or less than 4 mm, more preferably equal to or less than 3 mm, and further preferably equal to or less than 2.4 mm. By setting the intervals between the adjacent load portions to be equal to or less than 3 mm, the probability that any of the load portions comes into contact with the radial artery increases in many Japanese subjects. By setting the interval between the adjacent load portions to be equal to or less than 2.4 mm, the probability that any of the load portions comes into contact with the radial artery further increases in more Japanese subjects.

The plurality of extension portions 24 are fan-shaped or rectangular portions extending from the inside of the base portion 21 toward the beam 22 in a plan view. A gap (slit) having a size of about 1 mm is provided between each of the extension portions 24 and the beam 22. The extension portions 24 do not contribute to sensing of the pulse wave sensor 1, and thus need not be provided.

The length of the slit extending in the X direction and the length of the slit extending in the Y direction may be the same or need not be the same. For example, the length in the X direction of the slit defining the portion of the beam 22₁ located on the +X side relative to the load portion 23₁ may be the same as or different from the length in the Y direction of the slit defining the portion of the beam 22₄ located on the -Y side relative to the load portion 23₁.

The wire rod 30 is a cable for inputting and outputting electric signals between the pulse wave sensor 1 and the outside of the pulse wave sensor 1. The wire rod 30 may be a shielded cable, a flexible substrate, or the like. The wire rod 30 is not an essential component of the pulse wave sensor 1. The pulse wave sensor 1 may communicate with the outside with a method, such as wireless communication, without using the wire rod 30.

On the second surface 20n of the strain generating body 20, the pulse wave sensor 1 has a pair of strain gauges disposed on the beam 22, with the load portion 23 interposed between these strain gauges in a plan view, and another pair of strain gauges disposed on another beam 22 intersecting the beam 22 on which the former pair of strain gauges is disposed, with the load portion 23 interposed between these strain gauges in a plan view.

In the illustrated example, on the second surface 20n of the strain generating body 20, the pulse wave sensor 1 has a pair of strain gauges 100_{L1} and 100_{L2} disposed on the beam 22₁ extending in the X direction, with the load portion 23₁ interposed between the strain gauges 100_{L1} and 100_{L2} in a plan view. The pulse wave sensor 1 has another pair of strain gauges 100_{L3} and 100_{L4} disposed on the beam 22₄ intersecting the beam 22₁ on which the pair of strain gauges 100_{L1} and 100_{L2} are disposed, with the load portion 23₁ interposed between the strain gauges 100_{L3} and 100_{L4} in a plan view.

The strain gauges 100_{L1} and 100_{L2} detect a compressive strain of the beam 22₁ caused by pressing the load portion 23₁, and the strain gauges 100_{L3} and 100_{L4} detect a tensile strain of the beam 22₄ caused by pressing the load portion 23₁. The interval between the strain gauge 100_{L1} and the strain gauge 100_{L2}, both of which detect a compressive strain, is wider than the interval between the strain gauge 100_{L3} and the strain gauge 100_{L4}, both of which detect a tensile strain.

The pulse wave sensor 1 has, on the second surface 20n of the strain generating body 20, a pair of strain gauges 100_{C1} and 100_{C2} disposed on the beam 22₂ extending in the X direction, with the load portion 23₂ interposed between strain gauges 100_{C1} and 100_{C2} in a plan view. The pulse wave sensor 1 has another pair of strain gauges 100_{C3} and 100_{C4} disposed on the beam 22₄ intersecting the beam 22₂ on which the pair of strain gauges 100_{C1} and 100_{C2} are disposed, with the load portion 23₂ interposed between this other pair of strain gauges 100_{C3} and 100_{C4} in a plan view.

The strain gauges 100_{C1} and 100_{C2} detect a compressive strain of the beam 22₄ caused by pressing the load portion 23₂, and the strain gauges 100_{C3} and 100_{C4} detect a tensile strain of the beam 22₂ caused by pressing the load portion 23₂. The interval between the strain gauge 100_{C1} and the strain gauge 100_{C2}, both of which detect a compressive strain, is wider than the interval between the strain gauge 100_{C3} and the strain gauge 100_{C4}, both of which detect a tensile strain.

The pulse wave sensor 1 has, on the second surface 20n of the strain generating body 20, a pair of strain gauges 100_{R1} and 100_{R2} disposed on the beam 22₃ extending in the X direction, with the load portion 233 interposed between the strain gauges 100_{R1} and 100_{R2} in a plan view. The pulse wave sensor 1 has another pair of strain gauges 100_{R3} and 100_{R4} disposed on the beam 22₄ intersecting the beam 22₃ on which the pair of strain gauges 100_{R1} and 100_{R2} are disposed, with the load portion 23₂ interposed between the strain gauges 100_{R3} and 100_{R4} in a plan view.

The strain gauges 100_{R1} and 100_{R2} detect a compressive strain of the beam 22₃ caused by pressing the load portion 23₃, and the strain gauges 100_{R3} and 100_{R4} detect a tensile strain of the beam 22₄ caused by pressing the load portion 23₂. The interval between the strain gauge 100_{R1} and the strain gauge 100_{R2}, both of which detect a compressive strain, is wider than the interval between the strain gauge 100_{R3} and the strain gauge 100_{R4}, both of which detect a tensile strain.

By arranging the strain gauges 100 in this manner, it is possible to effectively detect a compressive strain and a tensile strain and obtain a large output through a full bridge.

The pulse wave sensor 1 is used by being fixed to the arm of a subject so that one or more load portions 23 among the plurality of load portions 23 come into contact with the radial artery of the subject. When a load is applied to any of the load portions 23 in accordance with a pulse wave of a subject and the beam 22 is elastically deformed, the resistance value of the resistor of the strain gauge 100 changes. The pulse wave sensor 1 can detect a pulse wave based on a change in the resistance value of the resistor of the strain gauge 100 caused by the deformation of the beam 22. A pulse wave is output as a periodic change in voltage from a measurement circuit connected to the electrodes of the strain gauge 100, for example.

A simulation of an amount of strain of the beam caused by pressing each load portion was performed for the pulse wave sensor 1 having the shape illustrated in FIGS. 1 and 2. In the strain generating body 20, the maximum length in the X direction in FIGS. 1 and 2 was 15 mm, the maximum length in the Y direction was 25 mm, and the thickness t was 0.1 mm. Each load portion 23 was circular with a diameter of 2 mm in a plan view and a height of 0.1 mm. The intervals between the adjacent load portions 23 were set to 3 mm. Although the intervals between the adjacent load portions 23 were set to 3 mm as an example here, it is expected that the same result is obtained even in the case where the intervals are other than 3 mm.

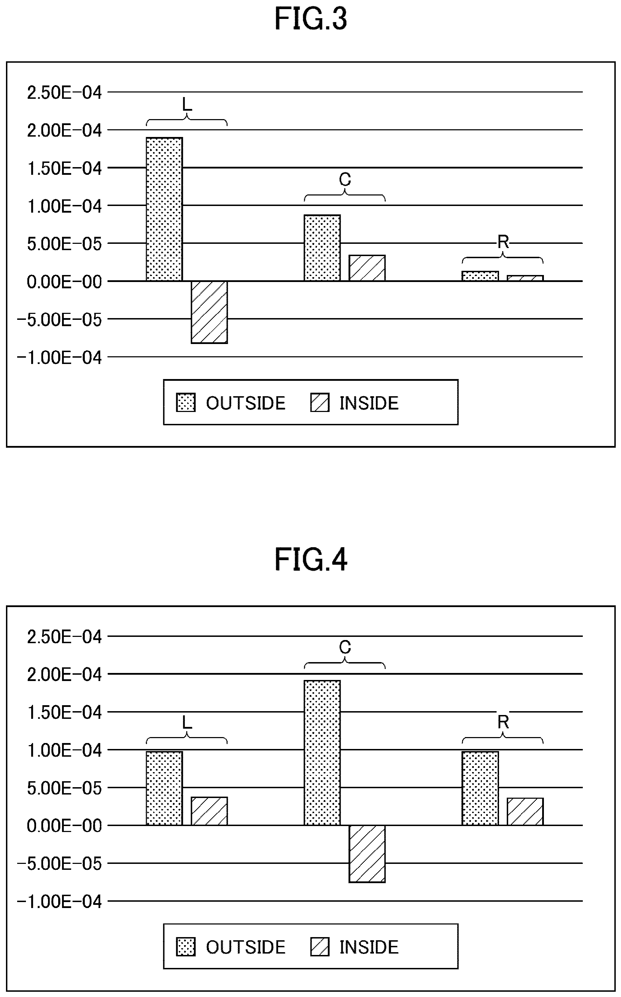

FIG. 3 illustrates a simulation result (1) of an amount of strain in a beam caused by pressing each load portion. FIG. 4 illustrates a simulation result (2) of an amount of strain in a beam caused by pressing each load portion. FIG. 3 illustrates an amount of strain when only the load portion 23₁ is pressed, and FIG. 4 illustrates an amount of strain when only the load portion 23₂ is pressed.

In FIGS. 3 and 4, the "L outside" indicates an amount of strain obtained by the strain gauges 100_{L1} and 100_{L2}, and the "L inside" indicates an amount of strain obtained by the strain gauges 100_{L3} and 100_{L4}. The "C outside" indicates an amount of strain obtained by the strain gauges 100_{C1} and 100_{C2}, and the "C inside" indicates an amount of strain obtained by the strain gauges 100_{C3} and 100_{C4}. The "R outside" indicates an amount of strain obtained by the strain gauges 100_{R1} and 100_{R2}, and the "R inside" indicates an amount of strain obtained by the strain gauges 100_{R3} and 100_{R4}.

As illustrated in FIG. 3, in the case where only the load portion 23₁ is pressed, a largest amount of strain is obtained by the strain gauges 100_{L1}, 100_{L2}, 100_{L3}, and 100_{L4} disposed closest to the load portion 23₁ with respect to the X direction and the Y direction.

Since the load portion 23₁ and the load portions 23₂ and 23₃ are disposed on the same beam 22₄, the load portion 23₁ being pressed also affects the load portions 23₂ and 23₃ that are not directly pressed. Therefore, as illustrated in FIG. 3, an amount of strain is detected in the C outside and inside and the R outside and inside as well.

The load portion 23₂ is closer to the load portion 23₁ than to the load portion 23₃. Therefore, the amount of strain of C obtained by the strain gauges 100_{C1}, 100_{C2}, 100_{C3}, and 100_{C4} is larger than the amount of strain of R obtained by the strain gauges 100_{R1}, 100_{R2}, 100_{R3}, and 100_{R4}.

As illustrated in FIG. 4, in the case where only the load portion 23₂ is pressed, a largest amount of strain is obtained by the strain gauges 100_{C1}, 100_{C2}, 100_{C3}, and 100_{C4} disposed closest to the load portion 23₂ with respect to the X direction and the Y direction.

Since the load portion 23₂ and the load portions 23₁ and 23₃ are disposed on the same beam 22₄, the load portion 23₂ being pressed also affects the load portions 23₁ and 23₃ that are not directly pressed. Therefore, as illustrated in FIG. 4, an amount of strain is detected in the outside and inside L and the outside and inside R.

The distance between the load portion 23₂ and the load portion 23₁ is the same as the distance between the load portion 23₂ and the load portion 23₃. Therefore, the amount of strain of L obtained by the strain gauges 100_{L1}, 100_{L2}, 100_{L3}, and 100_{L4} is larger than the amount of strain of R obtained by the strain gauges 100_{R1}, 100_{R2}, 100_{R3}, and 100_{R4}.

The sum of the amounts of distortion of L, C, and R illustrated in FIG. 3 coincides with the sum of the amounts of distortion of L, C, and R illustrated in FIG. 4. Although a simulation result in the case where only the load portion 23₃ is pressed is not illustrated in FIG. 4, the simulation would look similar to FIG. 3 except that the amounts of strain of the outside and inside L are swapped for the amounts of strain of the outside and inside R.

As described above, in the pulse wave sensor 1, the amount of strain can be detected when any one of the plurality of load portions 23 is pressed. In other words, by fixing the pulse wave sensor 1 to the arm of the subject with the arrangement direction of the load portions 23 (the Y direction in FIGS. 1 and 2) directed in a direction substantially perpendicular to the direction in which the radial artery extends, any one or more of the plurality of load portions 23 can be easily brought into contact with the radial artery of a subject. Then, a constant amount of strain can be obtained even when any of the load portions 23 abuts on the radial artery of the subject. Thus, the position detection mechanism of the radial artery and the adjustment of the position are not required, the pulse wave sensor 1 can be easily fixed to the arm of the subject, and the pulse wave can be reliably detected. In other words, by using the pulse wave sensor 1, it is possible to shorten the time required for attaching the pulse wave sensor 1 to the subject and improve the measurement accuracy.

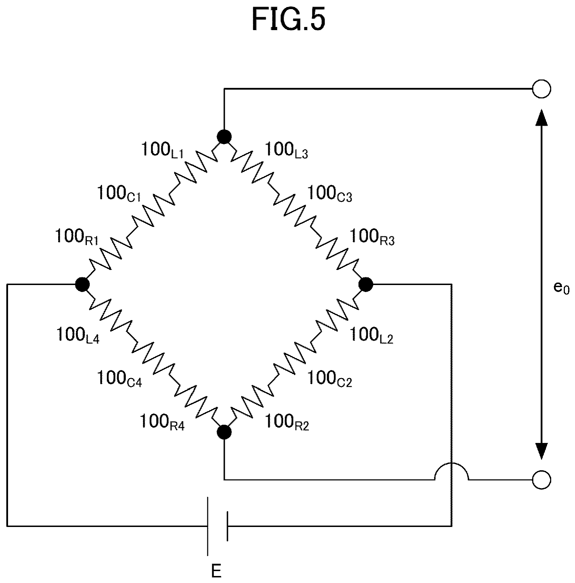

As illustrated in FIG. 5, the pulse wave sensor 1 can obtain a large output with a four-gauge method in which the strain gauges are connected in a full bridge.

In FIG. 5, all the strain gauges 100 illustrated in FIGS. 1 and 2 are connected to form one bridge circuit. Specifically, in the plan view illustrated in FIG. 1, the strain gauges 100 located closest to the respective load portions 23 with respect to the same direction are connected in series to form the respective sides of the bridge circuit.

For example, the strain gauge 100_{L1} located closest to the load portion 23₁ with respect to the -X direction, the strain gauge 100_{C1} located closest to the load portion 23₂ with respect to the -X direction, and the strain gauge 100_{R1} located closest to the load portion 23₃ with respect to the -X direction are connected in series to form the upper left side of the bridge circuit illustrated in FIG. 5.

The strain gauge 100_{L2} located closest to the load portion 23₁ with respect to the +X direction, the strain gauge 100_{C2} located closest to the load portion 23₂ with respect to the +X direction, and the strain gauge 100_{R2} located closest to the load portion 23₃ with respect to the +X direction are connected in series to form the upper right side of the bridge circuit illustrated in FIG. 5.

The strain gauge 100_{L3} located closest to the load portion 23₁ with respect to the -Y direction, the strain gauge 100_{C3} located closest to the load portion 23₂ with respect to the -Y direction, and the strain gauge 100_{R3} located closest to the load portion 23₃ with respect to the -Y direction are connected in series to form the lower right side of the bridge circuit illustrated in FIG. 5.

The strain gauge 100_{L4} located closest to the load portion 23₁ with respect to the +Y direction, the strain gauge 100_{C4} located closest to the load portion 23₂ with respect to the +Y direction, and the strain gauge 100_{R4} located closest to the load portion 23₃ with respect to the +Y direction are connected in series to form the lower left side of the bridge circuit illustrated in FIG. 5.

In FIG. 5, a direct current voltage E is supplied between a junction between the upper left side and the lower left side and a junction between the upper right side and the lower right side. As a result, the bridge circuit outputs a voltage e₀ between the junction of the upper left side and the upper right side and the junction of the lower left side and the lower right side.

The bridge connection may be performed inside or outside the housing 10. An interconnect board for bridge connection may be disposed inside the housing 10 as necessary.

### <Strain Gauge 100>

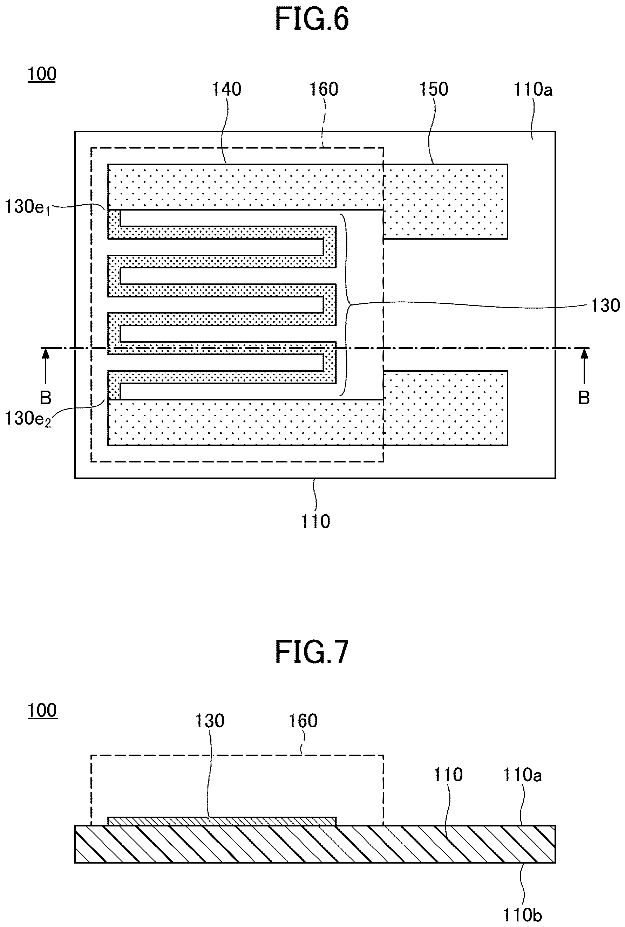

FIG. 6 is a plan view illustrating the strain gauge according to the first embodiment. FIG. 7 is a cross-sectional view (1) illustrating the strain gauge according to the first embodiment, and illustrates a cross-sectional view taken along line B-B in FIG. 6.

Referring to FIGS. 6 and 7, the strain gauge 100 includes a substrate 110, a resistor 130, an interconnect 140, electrodes 150, and a cover layer 160. In other words, the strain gauge 100 has the resistor 130 as a detection element. The cover layer 160 may be provided as necessary. In FIGS. 6 and 7, only the outer edge of the cover layer 160 is indicated by a broken line for convenience. First, each part constituting the strain gauge 100 will be described in detail.

In the description of the strain gauge with reference to FIGS. 6 through 8, the definitions of the upper surface and the lower surface are different from those in the other drawings. Specifically, in FIGS. 6 through 8, for convenience, in the strain gauge 100, the side of the substrate 110 on which the resistor 130 is provided is referred to as the "upper side", and the side on which the resistor 130 is not provided is referred to as the "lower side". A surface located on the upper side of each portion is referred to as an "upper surface", and a surface located on the lower side of each portion is referred to as a "lower surface". However, the strain gauge 100 can be used in an upside-down state. The strain gauge 100 may also be positioned at any angle. A "plan view" refers to viewing an object in a normal direction from the upper side to the lower side with respect to the upper surface 110a of the substrate 110. A "planar shape" refers to the shape of the object when the object is viewed in the normal direction. The strain gauge 100 is attached to the second surface 20n of the strain generating body 20 in such a manner that the substrate 110 faces the second surface 20n of the strain generating body 20.

The substrate 110 is a member serving as a base layer for forming the resistor 130 and the like. The substrate 110 has flexibility. The thickness of the substrate 110 is not particularly limited, and may be determined as appropriate according to the intended use of the strain gauge 100, and the like. For example, the thickness of the substrate 110 may be about 5 µm to 500 µm. In view of the transmission of strain from the second surface 20n of the strain generating body 20 to the sensing portion and the dimensional reliability against environmental changes, the thickness of the substrate 110 is preferably in the range of 5 µm to 200 µm. From the viewpoint of insulation properties, the thickness of the substrate 110 is preferably 10 µm or more.

The substrate 110 is formed of an insulating resin film made of an insulating resin, such as a polyimide (PI) resin, an epoxy resin, a polyether ether ketone (PEEK) resin, a polyethylene naphthalate (PEN) resin, a polyethylene terephthalate (PET) resin, a polyphenylene sulfide (PPS) resin, a liquid crystal polymer (LCP) resin, or a polyolefin resin. The film refers to a member having a thickness of approximately 500 µm or less and flexibility.

In the case where the substrate 110 is formed of an insulating resin film, the insulating resin film may contain a filler, an impurity, or the like. For example, the substrate 110 may be formed of an insulating resin film containing a filler, such as silica or alumina.

Examples of the material other than resins of the substrate 110 include crystalline materials, such as SiO₂, ZrO₂ (including YSZ), Si, Si₂N₃, Al₂O₃ (including sapphire), ZnO, and perovskite-based ceramics (CaTiO₃ and BaTiO₃). In addition to the crystalline materials described above, amorphous glass or the like may be used as the material of the substrate 110. As the material of the substrate 110, a metal, such as aluminum, an aluminum alloy (duralumin), or titanium, may be used. In the case of using the substrate 110 made of metal, an insulating film is provided so as to cover the upper surface 110a.

The resistor 130 is a thin film formed in a predetermined pattern on the upper side of the substrate 110. In the strain gauge 100, the resistor 130 is a sensitive part that receives a strain and causes a resistance change. The resistor 130 may be directly formed on the upper surface 110a of the substrate 110, or may be formed on the upper surface 110a of the substrate 110 via another layer. In FIG. 6, the resistor 130 is illustrated by a dotted pattern with a high density for convenience.

The resistor 130 has a structure in which a plurality of elongated portions are arranged at predetermined intervals with the longitudinal direction of the elongated portions directed in the same direction (the direction of line B-B in the example of FIG. 6), and the end portions of the adjacent elongated portions are alternately connected to each other and are folded back in a zigzag manner as a whole. The longitudinal direction of the plurality of elongated portions is a grid direction, and the direction perpendicular to the grid direction is a grid-width direction (the direction perpendicular to line B-B in the example of FIG. 6).

One end portion in the longitudinal direction of each of the two elongated portions located on the outermost side in the grid-width direction is bent in the grid-width direction to form end portions 130ₑ₁ and 130ₑ₂ of the resistor 130 in the grid-width direction. The end portions 130ₑ₁ and 130ₑ₂ of the resistor 130 in the grid-width direction are electrically connected to the electrodes 150 via the interconnect 140. In other words, the interconnect 140 electrically connects the end portions 130ₑ₁ and 130ₑ₂ of the resistor 130 in the grid-width direction to the electrodes 150.

The resistor 130 may be formed of, for example, a material containing Cr (chromium), a material containing Ni (nickel), or a material containing both Cr and Ni. In other words, the resistor 130 can be formed of a material containing at least one of Cr and Ni. Examples of the material containing Cr include a Cr composite film. Examples of the material containing Ni include Cu-Ni (copper nickel). Examples of the material containing both Cr and Ni include Ni-Cr (nickel chromium).

Herein, the Cr composite film is a film in which Cr, CrN, Cr₂N, and the like are mixed. The Cr composite film may contain inevitable impurities, such as chromium oxide.

The thickness of the resistor 130 is not particularly limited, and may be determined as appropriate according to the intended use of the strain gauge 100, and the like. For, example, the thickness of the resistor 130 may be about 0.05 µm to 2 µm. In particular, in the case where the thickness of the resistor 130 is 0.1 µm or greater, the crystallinity of the crystal constituting the resistor 130 (for example, the crystallinity of α-Cr) is improved. In the case where the thickness of the resistor 130 is equal to or less than 1 µm, (i) cracks in the film and (ii) warpage of the film from the substrate 110, which are caused by the internal stress of the film constituting the resistor 130, are reduced.

In consideration of making the transverse sensitivity less likely to occur and taking measures against disconnection, the width of the resistor 130 is preferably equal to or greater than 10 µm and equal to or less than 100 µm. More specifically, the width of the resistor 130 is preferably equal to or greater than 10 µm and equal to or less than 70 µm, and more preferably equal to or greater than 10 µm and equal to or less than 50 µm.

For example, when the resistor 130 is a Cr composite film, the stability of the gauge characteristics can be improved by using α-Cr (alpha chromium), which is a stable crystal phase, as a main component. For example, when the resistor 130 is a Cr composite film, the resistor 130 contains α-Cr as a main component, and thus the gauge factor of the strain gauge 100 can be 10 or more, and the gauge factor temperature coefficient TCS and the temperature coefficient of resistance TCR can be within the range of -1000 ppm/°C to +1000 ppm/°C. Herein, the "main component" means a component that accounts for 50% by weight or more of all substances constituting the resistor. From the viewpoint of improving the gauge characteristics, the resistor 130 preferably contains α-Cr in an amount of 80% by weight or more. Furthermore, from the same viewpoint, the resistor 130 more preferably contains α-Cr in an amount of 90% by weight or more. α-Cr is Cr having a bcc structure (body-centered cubic lattice structure).

In the case where the resistor 130 is a Cr composite film, the Cr composite film preferably contains 20% by weight or less of CrN and Cr₂N. In the case where the Cr composite film contains 20% by weight or less of CrN and Cr₂N, a decrease in the gauge factor of the strain gauge 100 can be suppressed.

The ratio of Cr₂N to CrN in the Cr composite film is preferably equal to or greater than 80% by weight and less than 90% by weight, more preferably equal to or greater than 90% by weight and less than 95%, with respect to the total weight of CrN and Cr₂N. Since Cr₂N has a semiconducting nature, the decrease in TCR (negative TCR) becomes more significant in the case where the ratio of Cr₂N to CrN in the Cr composite film to equal to or greater than 90% by weight and less than 95% by weight. Furthermore, in the case where the ratio of the Cr₂N to CrN to equal to or greater than 90% by weight and less than 95% by weight, the formation of ceramics in the resistor 130 can be reduced, and brittle fracture of the resistor 130 can be made less likely to occur.

CrN has an advantage of being chemically stable. By including a larger amount of CrN in the Cr composite film, the possibility of generating unstable N can be reduced, and thus a stable strain gauge can be obtained. Herein, "unstable N" means a small amount of N₂ or atomic N that may be present in the Cr composite film. These unstable N atoms may escape from the film depending on the external environment (for example, a high temperature environment). When unstable N escapes from the film, the film stress of the Cr composite film may change.

In the strain gauge 100, in the case where a Cr composite film is used as the material of the resistor 130, high sensitivity and miniaturization can be realized. For example, the output of a conventional strain gauge is about 0.04 mV/2V, whereas an output of 0.3 mV/2V or more can be obtained in the case where a Cr composite film is used as the material of the resistor 130. The size (gauge length × gauge width) of the conventional strain gauge is about 3 mm × 3 mm, whereas the size (gauge length × gauge width) of the resistor 130 using the Cr composite film as the material can be reduced to about 0.3 mm × 0.3 mm.

The interconnect 140 is provided over the substrate 110. The interconnect 140 is electrically connected to the resistor 130 and the electrodes 150. The interconnect 140 is not limited to a linear shape, and may have any pattern. The interconnect 140 can have any width and any length. In FIG. 6, the interconnect 140 is illustrated by a dotted pattern having a density lower than that of the resistor 130 for convenience.

The electrodes 150 are provided on the substrate 110. The electrodes 150 are electrically connected to the resistor 130 via the interconnect 140. The electrodes 150 are formed in a substantially rectangular shape with a width greater than that of the interconnect 140 in a plan view. The electrodes 150 are a pair of electrodes for outputting a change in the resistance value of the resistor 130 caused by a strain to the outside. For example, a lead wire for external connection is bonded to the electrode 150. A metal layer having low resistance, such as copper, or a metal layer having good solderability, such as gold, may be stacked on the upper surface of the electrode 150. The resistor 130, the interconnect 140, and the electrodes 150 are denoted by different reference numerals for convenience, but they can be integrally formed of the same material in the same process. In FIG. 6, the electrodes 150 are illustrated in a dotted pattern having the same density as the interconnect 140 for convenience.

The cover layer 160 (protection layer) is provided on the upper surface 110a of the substrate 110 as necessary so as to cover the resistor 130 and the interconnect 140 and expose the electrodes 150. Examples of the material of the cover layer 160 include insulating resins, such as PI resin, epoxy resin, PEEK resin, PEN resin, PET resin, PPS resin, and composite resin (for example, silicone resin and polyolefin resin). The cover layer 160 may contain a filler or a pigment. The thickness of the cover layer 160 is not particularly limited and may be appropriately selected depending on the intended purpose. For example, the thickness of the cover layer 160 may be about 2 µm to 30 µm. By providing the cover layer 160, it is possible to suppress an occurrence of mechanical damage or the like in the resistor 130. The provision of the cover layer 160 can protect the resistor 130 from moisture and the like.

### <Method for Manufacturing Strain Gauge 100>

In the strain gauge 100 according to the present embodiment, the resistor 130, the interconnect 140, the electrodes 150, and the cover layer 160 are formed on the substrate 110. Another layer (such as a functional layer described later) may be formed between the substrate 110 and the layers of these members.

Hereinafter, a method for manufacturing the strain gauge 100 will be described. In order to manufacture the strain gauge 100, first, the substrate 110 is prepared, and a metal layer (referred to as a "metal layer A" for the sake of simplicity) is formed on the upper surface 110a of the substrate 110. The metal layer A is a layer that is finally patterned to become the resistor 130, the interconnect 140, and the electrodes 150. Therefore, the material and thickness of the metal layer A are the same as the material and thickness of the resistor 130, the interconnect 140, and the electrodes 150 described above.

The metal layer A can be formed by, for example, a magnetron sputtering method using a raw material capable of forming the metal layer A as a target. The metal layer A may be formed by a reactive sputtering method, a vapor deposition method, an arc ion plating method, a pulse laser deposition method, or the like instead of the magnetron sputtering method. After the metal layer A is formed on the upper surface 110a of the substrate 110, the metal layer A is patterned into the same planar shape as the resistor 130, the interconnect 140, and the electrodes 150 in FIG. 6 by a known photolithography method.

The metal layer A may be formed after an underlayer is formed on the upper surface 110a of the substrate 110. For example, a functional layer having a predetermined thickness may be vacuum-deposited on the upper surface 110a of the substrate 110 by a conventional sputtering method. By providing the base layer in this manner, the gauge characteristics of the strain gauge 100 can be stabilized.

In the present application, the "functional layer" refers to a layer having a function of promoting crystal growth of at least the metal layer A (resistor 130) which is located above the functional layer. Preferably, the functional layer further has a function of preventing oxidation of the metal layer A due to oxygen and moisture contained in the substrate 110 and a function of improving adhesion between the substrate 110 and the metal layer A. The functional layer may further have other functions.

The insulating resin film constituting the substrate 110 may contain oxygen or moisture, and Cr may form a self-oxidized film. Therefore, particularly when the metal layer A contains Cr, it is preferable to form a functional layer having a function of preventing oxidation of the metal layer A.

By providing the functional layer under the metal layer A in this manner, the crystal growth of the metal layer A can be promoted, and the metal layer A having a stable crystal phase can be produced. As a result, the stability of the gauge characteristics of the strain gauge 100 is improved. The material constituting the functional layer is diffused into the metal layer A, and thus the gauge characteristics of the strain gauge 100 are improved.

Examples of the material of the functional layer include metals selected from the group consisting of chromium (Cr), titanium (Ti), vanadium (V), niobium (Nb), tantalum (Ta), nickel (Ni), yttrium (Y), zirconium (Zr), hafnium (Hf), silicon (Si), carbon (C), zinc (Zn), copper (Cu), bismuth (Bi), iron (Fe), molybdenum (Mo), tungsten (W), ruthenium (Ru), rhodium (Rh), rhenium (Re), osmium (Os), iridium (Ir), platinum (Pt), palladium (Pd), silver (Ag), gold (Au), cobalt (Co), manganese (Mn), and aluminum (Al). One type of the metal selected from the group or two or more types of the metal selected from the group can be used. An alloy of any metals selected from the group or a compound of any metals selected from the group may also be used.

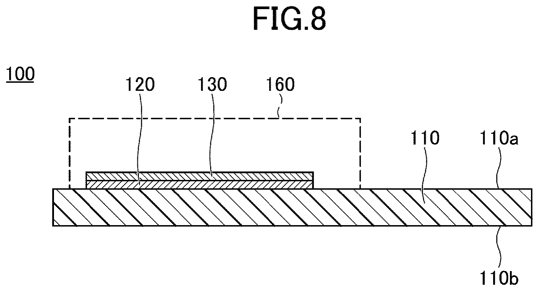

FIG. 8 is a cross-sectional view (2) illustrating the strain gauge according to the first embodiment. FIG. 8 illustrates a cross-sectional shape of the strain gauge 100 in the case where a functional layer 120 is provided as a base layer of the resistor 130, the interconnect 140, and the electrodes 150.

The planar shape of the functional layer 120 may be patterned to be substantially the same as the planar shape of the resistor 130, the interconnect 140, and the electrodes 150, for example. However, the planar shapes of the functional layer 120, the resistor 130, the interconnect 140, and the electrodes 150 need not be substantially the same. For example, in the case where the functional layer 120 is formed of an insulating material, the functional layer 120 may be patterned into a shape different from the planar shape of the resistor 130, the interconnect 140, and the electrode 150. In this case, the functional layer 120 may be formed in a solid shape in a region where the resistor 130, the interconnect 140, and the electrodes 150 are formed, for example. Alternatively, the functional layer 120 may be formed in a solid shape on the entire upper surface of the substrate 110.

After the resistor 130, the interconnect 140, and the electrodes 150 are formed, the cover layer 160 is formed on the upper surface 110a of the substrate 110 as necessary. The cover layer 160 covers the resistor 130 and the interconnect 140, but the electrodes 150 may be exposed from the cover layer 160. For example, the cover layer 160 can be formed by laminating a semi-cured thermoset insulating plastic film on the upper surface 110a of the substrate 110 so as to cover the resistor 130 and the interconnect 140 and expose the electrodes 150, and then heating and curing the insulating plastic film. The strain gauge 100 is completed by the above steps.

The preferred embodiments and the like have been described above in detail. However, the pulse wave sensor according to the present disclosure is not limited to the above-described embodiments, modified examples, and the like. For example, various modifications and substitutions can be made to the pulse wave sensor according to the above-described embodiment and the like without departing from the scope of the claims.

For example, in the above embodiment, a single beam 22₄ is provided on which the strain gauges 100 for detecting a tensile strain, which are denoted by the reference signs L, C, and R in FIG. 1, are disposed. However, three separate beams extending in the Y direction may be provided for the strain gauges 100 for detecting a tensile strain, which are denoted by the reference signs L, C, and R in FIG. 1. In this case, an amount of strain can be detected by providing one bridge circuit for the four strain gauges 100 denoted by the reference sign L in FIG. 1, one bridge circuit for the four strain gauges 100 denoted by the reference sign C, and one bridge circuit for the four strain gauges 100 denoted by the reference sign R.

The number of the load portions 23 is not limited to three, and may be two or more. Of course, the number of the load portions 23 may be four or more. The arrangement of the load portions 23 is not limited to one row, and may be a plurality of rows.

In the above embodiment, the pulse wave sensor 1 and the like detect a pulse wave, but the detection target of the pulse wave sensor 1 is not limited to a pulse wave. The pulse wave sensor 1 and the like can detect, for example, a pulse pressure, a blood pressure, a pulse, an oxygen level, and the like.

This international application claims priority based on Japanese Patent Application No. 2023-040008 filed on March 14, 2023, and the entire contents of Japanese Patent Application No. 2023-040008 are incorporated herein by reference.

### REFERENCE SIGNS LIST

1 Pulse wave sensor
10 Housing
20 Strain generating body
20m First surface
20n Second surface
21 Base portion
22₁, 22₂, 22₃, 22₄ Beams
23₁, 23₂, 23₃ Load portions
24 Extension portion
30 Wire rod
100_{L1}, 100_{L2}, 100_{L3}, 100_{L4}, 100_{C1}, 100_{C2}, 100_{C3} 100_{C4}, 100_{R1}, 100_{R2}, 100_{R3}, 100_{R4} Strain gauges
110 Substrate
110a Upper surface
130 Resistor
140 Interconnect
150 Electrodes
160 Cover layer
130ₑ₁, 130ₑ₂ End portions

## Claims

1. A pulse wave sensor, comprising:
a strain generating body including a first surface and a second surface located on an opposite side of the first surface;
a plurality of beams provided on the strain generating body and intersecting;
a load portion disposed in a region on the first surface, the region being defined by an intersection of the plurality of beams on the first surface, the region being one or more;
a first pair of strain gauges disposed on one beam of the plurality of beams on the second surface, with the load portion interposed between the first pair of strain gauges in a plan view; and
a second pair of strain gauges disposed on another beam of the plurality of beams, the another beam intersecting the one beam on which the first pair of strain gauges are disposed, on the second surface, with the load portion interposed between the second pair of strain gauges in a plan view, wherein
a pulse wave is detected based on a change in resistance value of each strain gauge of the first pair of strain gauges and the second pair of strain gauges caused by deformation of the plurality of beams.

2. The pulse wave sensor according to claim 1, wherein
the first pair of strain gauges or the second pair of strain gauges detects a compressive strain of the plurality of beams caused by the load portion being pressed, and
the first pair of strain gauges or the second pair of strain gauges that does not detect the compressive strain detects a tensile strain of the beams caused by the load portion being pressed.

3. The pulse wave sensor according to claim 2, wherein
an interval between the first pair of strain gauges or the second pair of strain gauges that detects the compressive strain is wider than an interval between the first pair of strain gauges or the second pair of strain gauges that detects the tensile strain.

4. The pulse wave sensor according to claim 2 or 3, wherein
the plurality of beams include a first beam and a plurality of second beams intersecting the first beam.

5. The pulse wave sensor according to claim 4, wherein
the first beam extends in a first direction, and
the plurality of second beams intersecting the first beam extend in a second direction perpendicular to the first direction.

6. The pulse wave sensor according to claim 5, wherein
the plurality of second beams intersecting the first beam include three or more beams arranged at equal intervals.

7. The pulse wave sensor according to claim 5 or 6, wherein
the strain gauges that detect the compressive strain are disposed on each of the plurality of second beams extending in the second direction, and
the strain gauges that detect the tensile strain are disposed on the first beam extending in the first direction.

8. The pulse wave sensor according to any one of claims 5 to 7, wherein
the strain generating body has a shape in which a longitudinal direction extends in the first direction and a lateral direction extends in the second direction.

9. The pulse wave sensor according to any one of claims 4 to 8, wherein
all the strain gauges of the first and second pairs are connected to form one bridge circuit, and
any of the strain gauges of the first and second pairs located closest to the load portion with respect to a same direction in a plan view is connected in series to form each side of the bridge circuit.

10. The pulse wave sensor according to any one of claims 1 to 9, wherein
an interval between adjacent load portions is equal to or greater than 1 mm and equal to or less than 3 mm, the adjacent load portions being the load portion.

11. The pulse wave sensor according to claim 10, wherein
the interval is equal to or greater than 1 mm and equal to or less than 2.4 mm.
